# EUROPÄISCHE PATENTSCHRIFT

(11) **EP 2 285 448 B1**
(45) Veröffentlichungstag und Bekanntmachung des Hinweises auf die Patenterteilung: **06.11.2019**
(21) Anmeldenummer: 09761445.7
(22) Anmeldetag: 08.06.2009
(51) Int. Cl.: A61N 5/10

(54) **DEPOSITION EINER SOLLDOSISVERTEILUNG IN EINEM ZYKLISCH BEWEGTEN ZIELGEBIET**
DEPOSITION OF AN INTENDED DOSE DISTRIBUTION IN A CYCLICALLY MOVED TARGET AREA
DÉPÔT D'UNE DISTRIBUTION DE DOSES DE CONSIGNE DANS UNE ZONE CIBLE PRÉSENTANT DES MOUVEMENTS CYCLIQUES

(30) Priorität: 09.06.2008 DE 102008027485
(43) Veröffentlichungstag der Anmeldung: 23.02.2011
(73) Patentinhaber: GSI Helmholtzzentrum für Schwerionenforschung GmbH, 64291 Darmstadt (DE)
(72) Erfinder: BERT, Christoph, 63741 Aschaffenburg (DE); RIETZEL, Eike, 64331 Weiterstadt (DE)
(74) Vertreter: Mergel, Volker
(86) Internationale Anmeldenummer: PCT/EP2009/004096
(87) Internationale Veröffentlichungsnummer: WO 2009/149882

(56) Entgegenhaltungen:
- EP-A- 1 905 481
- WO-A-00/48680
- BERT CHRISTOPH: "Bestrahlungsplanung für bewegte Zielvolumina in der Tumortherapie mit gescanntem Kohlenstoffstrahl" INTERNET CITATION, [Online] 3. Februar 2006 (2006-02-03), XP002481370 Gefunden im Internet: URL:http://elib.tu-darmstadt.de/diss/00064 8> [gefunden am 2008-06-24]
- PHILLIPS M H ET AL: "Effects of respiratory motion on dose uniformity with a charged particle scanning method" PHYSICS IN MEDICINE AND BIOLOGY, TAYLOR AND FRANCIS LTD. LONDON, GB, Bd. 37, Nr. 1, 1. Januar 1992 (1992-01-01), Seiten 223-233, XP020021933 ISSN: 0031-9155

## Beschreibung

Die vorliegende Erfindung bezieht sich auf ein Verfahren und auf eine Bestrahlungsvorrichtung zur Deposition einer Solldosisverteilung in einem zyklisch bewegten Zielgebiet durch Mehrfachbestrahlung mit einem Rasterpunkte anfahrenden Strahl, sowie auf ein Verfahren zur Bestimmung von Steuerungsparametern für die Bestrahlungsvorrichtung.

Die Bestrahlung eines Zielgebiets mit einem verschiedene Punkte anfahrenden Strahl (beam scanning) ist an sich bekannt. So werden etwa bei der Bestrahlung von Tumoren beispielsweise Partikelstrahlen, insbesondere mit Protonen, α-Teilchen und Kohlenstoffkernen, eingesetzt. Es werden Teile des Zielgebiets sequenziell nacheinander mit dem Strahl angefahren (scanning).

Beispielsweise zeigt die Dissertation von Herrn Prof. Dr. Bert, "Bestrahlungsplanung für bewegte Zielvolumina in der Tumortherapie mit gescanntem Kohlenstoffstrahl", die Grundlage für die zweiteilige Fassung des Anspruchs 1 ist, eine solche Bestrahlung.

Solche Partikelstrahlen sind für die Bestrahlung räumlicher Zielgebiete besonders vorteilhaft, da sie zu ihrem Ende hin ein Maximum in der Energiedeposition durchlaufen (Bragg-Peak). So können etwa auch eingebettete räumliche Strukturen effektiv bestrahlt werden, ohne die einbettende Umgebung allzu stark zu schädigen. Oft werden räumliche Zielgebiete schichtweise bestrahlt, wobei die die Eindringtiefe bestimmende Strahlenergie je Schicht konstant gewählt wird (Isoenergieschicht). Die Erfindung betrifft grundsätzlich auch Ausführungsformen, bei denen der Strahl durch elektromagnetische Wellen gebildet wird. Weiter betrifft die Erfindung grundsätzlich auch Ausführungsformen zur Bestrahlung eines flächigen Zielgebiets.

Der Strahl hat üblicherweise einen kleineren Querschnitt als das Zielgebiet. Um das Zielgebiet insgesamt bestrahlen zu können, fährt der Strahl hintereinander mehrere Rasterpunkte in einem Zielraster an. Die hintereinander angefahrenen Rasterpunkte werden auch als Pfad (scanpath) bezeichnet. Umfasst ein Überlapp zwischen dem Zielraster und dem Zielgebiet das gesamte Zielgebiet, so kann durch sukzessives Anfahren der Rasterpunkte das Zielgebiet insgesamt bestrahlt werden. Insofern wird auch von einer Unterteilung des Zielgebiets in mehrere Rasterpunkte gesprochen.

Die beschriebenen Maßnahmen sind bei verschiedenen Scanverfahren verwendbar.

Bei dem so genannten Spotscanverfahren verweilt der Partikelstrahl an jedem Rasterpunkt für eine vorbestimmte Zeit und/oder deponiert an jedem Rasterpunkt eine vorbestimmte Anzahl Partikel und wird ausgeschaltet, während Ablenkmagnete etc. auf einen nächsten Rasterpunkt eingestellt werden.

Bei dem so genannten Rasterscan-Verfahren verweilt der Partikelstrahl an jedem Rasterpunkt während einer vorbestimmten Zeitdauer oder deponiert an jedem Rasterpunkt eine vorbestimmte Anzahl Partikel, wird aber zwischen den Rasterpunkten nicht oder nicht immer ausgeschaltet.

Bei so genannten kontinuierlichen Scanverfahren bilden die Rasterpunkte zusammenhängende Linien, bilden also kontinuierliche (oder quasikontinuierliche) Mengen, wobei ihre Anzahl etwa abzählbar unendlich sein kann. Der Partikelstrahl wird bei einem kontinuierlichen Scanverfahren zumindest innerhalb einer Linie bzw. Zeile in einer Isoenergieschicht kontinuierlich abgelenkt und überstreicht die Rasterpunkte, ohne an einzelnen Orten zu verweilen.

Mit einer Tiefenmodulationsvorrichtung kann auch ein kontinuierliches Scanverfahren durchgeführt werden, bei dem kontinuierlich die Eindringtiefe des Partikelstrahls moduliert wird.

Der Pfad kann etwa innerhalb einer isoenergetischen Schicht verlaufen, indem der Strahl lediglich in seiner Verlaufsrichtung, also lateral, abgelenkt wird, oder er kann etwa auch zwischen isoenergetischen Schichten verlaufen, indem die Energie des Strahls geändert wird.

Üblicherweise soll, etwa bei der Bestrahlung eines Tumors, eine bestimmte Verteilung der Dosis, also eine Solldosisverteilung, über das Zielgebiet erzielt werden. Die Solldosisverteilung wird etwa als deponierte Energie pro Flächeneinheit beziehungsweise pro Volumeneinheit quantifiziert. Verbreitet ist eine Angabe einer Dosis in Joule pro Kilogramm (Gray).

Bei der an sich ebenfalls bekannten Mehrfachbestrahlung mit einem verschiedene Punkte anfahrenden Strahl (rescanning) wird die Solldosis während einer Sitzung in mehreren, gegebenenfalls durch kurze Pausen getrennten, Durchgängen (scans) hintereinander appliziert. Innerhalb der einzelnen Durchgänge werden dabei mehrere der Rasterpunkte, nicht notwendigerweise alle, sequenziell bestrahlt. Im Laufe einer Sitzung wird üblicherweise ein Großteil der Rasterpunkte beziehungsweise werden sämtliche Rasterpunkte und damit ein Großteil des Zielgebiets beziehungsweise das gesamte Zielgebiet mehrfach bestrahlt. Die für die Sitzung zu applizierende Gesamtdosis pro Rasterpunkt wird auf die einzelnen Durchgänge aufgeteilt, und zwar etwa gleichverteilt oder gegebenenfalls auch mit unterschiedlicher Gewichtung.

Oft sind die zu bestrahlenden Zielgebiete relativ zu der bestrahlenden Vorrichtung nicht in Bewegung. Bekannt ist aber auch das Bestrahlen eines sich zyklisch bewegenden Zielgebiets, etwa eines sich durch die Atmung oder aufgrund einer sonstigen Organbewegung einer Person bewegenden Tumors.

Zyklische Bewegungen umfassen etwa periodische und von einer mathematisch exakten Periodizität abweichende quasiperiodische Bewegungen. Insbesondere werden auch solche Bewegungen als zyklisch betrachtet, die zeitweise in einer Phase verweilen.

Der vorliegenden Erfindung liegt die Aufgabe zugrunde, ein vorteilhaftes Verfahren und eine vorteilhafte Vorrichtung zur Deposition einer Solldosisverteilung in einem zyklisch bewegten Zielgebiet durch Mehrfachbestrahlung mit einem Rasterpunkte anfahrenden Strahl anzugeben, sowie ein Verfahren zur Bestimmung von Steuerungsparametern für die Vorrichtung.

Diese Aufgabe wird gelöst durch das Verfahren gemäß Anspruch 1: Verfahren zur Deposition einer Solldosisverteilung in einem zyklisch bewegten Zielgebiet, welches nicht in einem Mensch oder Tier angeordnet ist, durch Mehrfachbestrahlung mit einem Rasterpunkte eines Zielrasters anfahrenden Strahl in eine Anzahl von zumindest zwei Durchgängen, bei dem in jedem der Durchgänge Rasterpunkte aufeinanderfolgend abgetastet bzw. angefahren werden. Es ist gekennzeichnet durch die Schritte: Desynchronisieren des Ablaufs der Bestrahlung und der zyklischen Bewegung des Zielgebiets durch Veränderung zumindest einer der folgenden Steuerungsparameter: Abtastgeschwindigkeit des Strahls; Zeitliche Verteilung der Durchgänge, wobei die Durchgänge zufällig verteilt werden oder wobei Pausenzeiten zwischen einzelnen Durchgängen variiert werden; zeitliche Verteilung des Bestrahlens von Schichten; zeitliche Verteilung des Bestrahlens von bestimmten Volumina innerhalb der einzelnen Durchgänge; Zyklusdauer der zyklischen Bewegung, wobei die Dauer des Zyklus zufällig geändert wird; Pfad des Strahls im Zielgebiet, und Aufteilen der Bestrahlung des Zielgebiets auf bis zu 30 Durchgänge, so dass lokale Fehldosierungen klein gehalten werden.

Bevorzugte Ausgestaltungen der Erfindung sind in abhängigen Ansprüchen angegeben und werden im Folgenden näher erläutert.

Die Erfindung beruht auf der Beobachtung, dass bei zyklisch bewegtem Zielgebiet die erzielte Dosisverteilung von der Solldosisverteilung abweichen kann; insbesondere treten lokale Über- und Unterdosierungen auf.

Der Erfindung liegt die Erkenntnis zugrunde, dass der zeitliche Ablauf der Bestrahlung, insbesondere das Anfahren der Rasterpunkte, und die zyklische Bewegung derart miteinander wechselwirken können, dass solche lokalen Fehldosierungen entstehen können. Anders ausgedrückt: Lokale Abweichungen von der Solldosierung können durch eine Interferenz bzw. Synchronisation zwischen dem Ablauf der Bestrahlung und der zyklischen Bewegung des Zielgebiets entstehen bzw. verstärkt werden.

Beispielsweise kann der Pfad des Strahls zeitweise gleichlaufend mit dem sich bewegenden Zielgebiet bewegt werden, was zu einer Überdosierung führt, oder zeitweise in Gegenrichtung, was zu einer Unterdosierung führt.

Weiter können etwa durch eine ungünstige Synchronisation zwischen der Abfolge der Durchgänge und der zyklischen Bewegung lokale Abweichungen von der Solldosisverteilung von Durchgang zu Durchgang verstärkt werden. Auch hier kann man von einem Gleichlauf zweier Bewegungen sprechen. Ergänzend zu oben bezeichnet der Begriff "Gleichlauf" auch ein zeitliches Folgen im Sinne der Wiederholung einer Fehldosierung in unterschiedlichen Durchgängen am gleichen Ort bzw. in der gleichen Region des Zielgebiets.

Der Erfindung liegt die Idee zugrunde, dass durch eine Desynchronisierung, man kann auch sagen Entkopplung, des Ablaufs der Bestrahlung und des Ablauf der zyklischen Bewegung, Abweichungen von der Solldosierung vermindert werden können. Durch die Desynchronisierung wird das Entstehen eines Gleichlaufs gestört oder sogar verhindert; so können lokale Fehldosierungen etwa durch statistisches Mitteln über die Durchgänge oder auch durch gezieltes Entgegenwirken vergleichsweise klein gehalten werden.

In anderen Worten: Der Ablauf der Mehrfachbestrahlung und die zyklische Bewegung werden nach der Erfindung in dem Sinne aufeinander abgestimmt beziehungsweise desynchronisiert, dass Fehldosierungen entgegengewirkt wird bzw. eine in einem der Durchgänge entstandene Fehldosierung sich insgesamt nur schwach, wenn überhaupt, auf die erzielte Dosisverteilung auswirkt.

Für das Verständnis der Erfindung kann es hilfreich sein, zu wissen, dass eine maximal tolerierbare Abweichung von der Solldosisverteilung festgelegt werden kann. Diese kann von Anwendung zu Anwendung unterschiedlich sein und wird etwa empirisch oder anhand von Modellrechungen bestimmt (vgl. unten); die entsprechende Angabe kann etwa in einer Tabelle im Speicher eines steuernden Rechners abgelegt sein (vgl. unten). Insbesondere bei Patientenbehandlungen kann die Berücksichtigung der Solldosis auch die biologisch effektive Dosis darstellen. Es ist nicht erforderlich, bei jeder Bestrahlung die tolerierbare Abweichung erneut anzugeben. Die Angabe muss auch nicht ausdrücklich quantitativ angegeben sein; es reicht etwa, wenn das Verfahren so ausgestaltet wird bzw. die das Verfahren bestimmenden Parameter so gewählt werden, dass die Abweichungen von der Solldosisverteilung kleiner sind als bspw. in vorangehenden Fällen, empirischen Studien, Referenzfällen oder in Modellrechnungen; Die Festlegung kann also auch durch die konkrete Ausgestaltung des Verfahrens erfolgen. In anderen Worten: Das "Festlegen" kann auch durch ein "Auslegen" der Bestrahlungsvorrichtung zur Unterschreitung eines maximal tolerierten Fehlers umgesetzt werden. Vorzugsweise betragen lokale Abweichungen von der Solldosisverteilung höchstens 30%. Zunehmend bevorzugt sind Abweichungen von höchstens 20%, 10%, 5% beziehungsweise sogar von nur 2% in der angegebenen Reihenfolge. Grundsätzlich ist es auch möglich, die Fehldosierung online zu bestimmen, um während der Bestrahlung die Parameter für das Bestrahlungsverfahren anzupassen.
Eine Desynchronisation des Ablaufs der Mehrfachbestrahlung und der zyklischen Bewegung kann durch verschiedene Maßnahmen erzielt werden. Solche Maßnahmen können etwa auf einer Einstellung und/oder Veränderung folgender Größen beruhen:
- Strahlintensität, und damit zusammenhängend, die Abtastgeschwindigkeit des Strahls;
- zeitliche Verteilung der Durchgänge, etwa bezogen auf den Beginn der jeweiligen Durchgänge;
- bei räumlichen Zielgebieten die zeitliche Verteilung des Bestrahlens von Schichten bzw. von bestimmten Volumina innerhalb der einzelnen Durchgänge, etwa bezogen auf den Beginn des Bestrahlens einer Schicht bzw. eines Volumens, etwa durch variierende Beschleunigerextraktion oder durch passive Energiemodulation;
- Anfangsphase und/oder Zyklusdauer der zyklischen Bewegung, soweit die Bewegung entsprechend beeinflusst werden kann;
- Pfad des Strahls im Zielgebiet, insbesondere innerhalb von Schichten bzw. Volumina innerhalb eines entsprechend unterteilten Zielgebiets.

Für das Verständnis der Erfindung ist es hilfreich zu wissen, dass die Bestrahlung in so viele Durchgänge aufgeteilt wird, dass lokale Abweichungen von der Solldosisverteilung höchstens den oben angegebenen Abweichungen entsprechen. Grundsätzlich gilt, dass je größer die Anzahl der Durchgänge, desto weniger wirkt sich eine in einem Durchgang entstandene Fehldosierung auf die Gesamtdosisverteilung aus. Fehldosierungen einzelner Durchgänge können sich ausmitteln beziehungsweise kompensieren.

Es ist ferner für das Verständnis der Erfindung hilfreich zu wissen, dass je nach Anwendung beziehungsweise zu bestrahlendem Ziel im Vorfeld etwa Versuche oder Modellrechnungen durchgeführt werden können, um die hinreichende Anzahl an Durchgängen zu bestimmen. Solche Versuche werden üblicherweise an so genannten Phantomen, etwa basierend auf einem Körper aus Plexiglas oder Wasser, durchgeführt; die an einem Phantom gewonnenen Ergebnisse lassen Schlüsse auf die für andere Ziele zu verwendenden Bestrahlungsparameter, insbesondere die Anzahl der Durchgänge, zu. Gegebenenfalls können auch Modellrechnungen für einzelne bzw. Gruppen von Patienten durchgeführt werden.

Mit Hilfe der Erfindung kann auch die Wechselwirkung zwischen einem Rasterpunkte anfahrenden Strahl und einer zyklischen Bewegung des Ziels besser verstanden werden, womit zur Verbesserung von Verfahren und Vorrichtungen für eine solche Mehrfachbestrahlung beigetragen werden kann; insbesondere können auch die die Mehrfachbestrahlung bestimmenden Parameter überprüft beziehungsweise angepasst werden. Neben der Bestrahlung von Personen oder Tieren ist auch die Bestrahlung von organischem Material überhaupt, insbesondere von Zellen, oder auch die Bestrahlung anorganischer Materialien, etwa von Kunststoffen, relevant. So kann die Erfindung bspw. auch zur Erforschung von Materialeigenschaften beitragen; es können etwa die oben erwähnten Phantome untersucht und verbessert werden. Es bietet sich an, mit Hilfe solcher Phantome und gegebenenfalls eingebetteter Detektoren auch die Bestrahlungsparameter zu überprüfen oder zu bestimmen.

Für das Verständnis der Erfindung ist es hilfreich zu wissen, dass zumindest eine Eigenschaft der Mehrfachbestrahlung von zumindest einer Eigenschaft der zyklischen Bewegung abhängig sein kann. Beispielsweise können die Mehrfachbestrahlung bestimmende Parameter an die die zyklische Bewegung charakterisierende Eigenschaften angepasst werden. So kann etwa eine Eigenschaft der Bewegung eines Organs, etwa der Atembewegung der Lunge und der umliegenden Organe einer Person, in ein Signal umgesetzt werden und dieses einer Steuervorrichtung zur Steuerung der Mehrfachbestrahlung zugeführt werden. Beispielsweise kann die Mehrfachbestrahlung von der mittleren Zyklusdauer oder der mittleren Amplitude der Bewegung abhängen.

So können etwa folgende Eigenschaften beziehungsweise Parameter der Mehrfachbestrahlung von zumindest einer der Eigenschaften der zyklischen Bewegung abhängig sein:
- Anzahl der Durchgänge in einer Sitzung;
- Strahlintensität während eines Durchgangs;
- Geschwindigkeit mit der der Strahl die Rasterpunkte innerhalb eines Durchgangs von Rasterpunkt zu Rasterpunkt wechselt (man spricht auch von Strahlgeschwindigkeit);
- Startzeitpunkte der einzelnen Durchgänge.

Die Eigenschaften der Bestrahlung können etwa auf die Zyklusdauer oder die momentane Phase abgestimmt werden.

Die Startzeitpunkte der einzelnen Durchgänge können etwa verteilt sein
- über einen bestimmten Abschnitt der zyklischen Bewegung, beispielsweise bei einer Atembewegung, etwa über einen Inspirationsabschnitt oder einen Exspirationsabschnitt,
- über einen ganzen Zyklus, etwa eine Atemperiode und
- über eine Zeit, die größer ist als eine Zyklusdauer.

Sie können beispielsweise so gewählt werden, dass eine Bestrahlung vor allem, insbesondere häufiger, in einem Zustand des zu bestrahlenden Objekts erfolgt, in dem die zyklische Bewegung stärker oder auch schwächer ausgeprägt ist als in einem anderen Zustand (vgl. unten).

So ein Zustand des zu bestrahlenden Ziels beziehungsweise eine Eigenschaft der zyklischen Bewegung kann dazu in ein Signal umgesetzt werden, welches etwa zur Steuerung der Startzeitpunkte, etwa der einzelnen Durchgänge, benutzt werden kann. Diese Startzeitpunkte können dabei etwa gleichmäßig oder zufällig verteilt werden.

Das Signal kann etwa mehrfach oder fortlaufend während der zyklischen Bewegung bestimmt werden (online). Das Signal kann aber auch im Rahmen einer Planungsphase vor der eigentlichen Bestrahlung überwacht und/oder bestimmt werden und hieraus eine spezifische Bewegung, etwa eine patientenspezifische Organbewegung, abgeleitet werden.

Es kann aber etwa auch ein Signal vorgegeben werden, mit dem auch Einfluss auf die zyklische Bewegung genommen wird (Instruktionssignal). So kann beispielsweise eine zu bestrahlende Person mit einem Signal instruiert werden, ihren Atemzyklus konstant zu halten oder eine Änderung in der Atmung vorzunehmen. So kann dieser Person etwa vorgegeben werden, wann sie einzuatmen und wann sie auszuatmen hat. In diesem Fall hängt die Wahl der Eigenschaften der Bestrahlung von dem Signal und damit mittelbar von den Eigenschaften der zyklischen Bewegung ab.

Vorzugsweise wird während der Mehrfachbestrahlung ein die Mehrfachbestrahlung bestimmender Parameter verändert. Dies kann in regelmäßigen Abständen geschehen; vorzugsweise geschieht dies zu zufälligen Zeitpunkten.

Es können etwa die während eines Durchgangs angewandte Strahlgeschwindigkeit und/oder Strahlintensität geändert werden. Weiter können etwa auch Pausenzeiten zwischen einzelnen Durchgängen variiert werden oder auch der Pfad des Strahls während einer Sitzung der Mehrfachbestrahlung variiert werden.

So eine Veränderung kann beispielsweise während einer Bestrahlungssitzung von Durchgang zu Durchgang erfolgen oder bei einer Bestrahlung mit mehreren Sitzungen von Sitzung zu Sitzung; im letzten Fall kann insbesondere auch die deponierte Strahlendosis und die Anzahl der Durchgänge pro Sitzung geändert werden. Bevorzugt ist eine Veränderung innerhalb der Sitzung.

Vorzugsweise wird zumindest ein Teil der Rasterpunkte in unterschiedlichen Durchgängen gezielt in unterschiedlichen Phasen der zyklischen Bewegung mit dem Strahl angefahren. Dies kann etwa durch den gezielten Beginn unterschiedlicher Durchgänge in unterschiedlichen Phasen der zyklischen Bewegung realisiert werden. Eine in einer der Phasen gegebene Fehldosierung wird so durch Bestrahlen in den anderen Phasen zumindest teilweise korrigiert werden. Besonders bevorzugt ist es, zumindest 50%, besser 80% noch besser 90% der Durchgänge in unterschiedlichen Phasen der zyklischen Bewegung zu beginnen. Vorzugsweise wird dazu die zyklische Bewegung überwacht, insbesondere die Bewegungsperiode oder Phase mehrfach oder kontinuierlich bestimmt.

Periodischen Bewegungen ist eine eindeutige Periodendauer, kurz Periode, zugeordnet. Bei quasiperiodischen Bewegungen lässt sich zumindest näherungsweise eine Periode angeben, etwa eine mittlere Periode über mehrere Zyklen oder auch eine typische Periode. Weist eine zyklische Bewegung von einer periodischen Bewegung abweichende, etwa bewegungslose, Zeitabschnitte auf, so kann man einer solchen Bewegung zumindest zeitweise näherungsweise eine Periode zuordnen, etwa durch Auswerten bestimmter Zeitabschnitte.

Zur Bestimmung einer Periode kann beispielsweise auf eine systemspezifische beziehungsweise personenspezifische Zeit abgestellt werden. So beträgt die Atemperiode eines erwachsenen Menschen in Ruhe typischerweise etwa vier Sekunden. Es kann aber auch etwa auf eine mittlere über mehrere Zyklen gemessene Periode zurückgegriffen werden. In einer bevorzugten Ausführungsform wird auf Werte größer 100% beispielsweise 140% der mittleren gemessenen oder empirischen oder personenspezifischen Periode zurückgegriffen.

Bei einer bevorzugten Ausführungsform wird die Periode vor und/oder auch während einer Bestrahlung, gegebenenfalls näherungsweise, bestimmt und zumindest ein Teil der Durchgänge wird über eine Zeit verteilt, welche zwischen 50% und 140%, besser zwischen 75% und 140% der bestimmten Periode entspricht. Auch andere Zeiten ab 50% bzw. 75% können vorteilhaft sein; sowie Zeiten bis 100%. Vorzugsweise werden sämtliche Durchgänge über die genannten Zeitintervalle beziehungsweise das ausgewählte Zeitintervall verteilt. Auch eine Verteilung über zumindest zwei Zyklen oder über eine andere wählbare, gegebenenfalls auch längere, Zeit kann vorteilhaft sein.

So kann eine Verteilung über verschiedene Phasen gewährleistet werden. Insbesondere ist es bevorzugt, die Durchgänge möglichst gleichmäßig über die angegebenen Zeiten zu verteilen. Die Durchgänge können jedoch auch zufällig verteilt werden. Zwei wichtige Parameter, die die Verteilung der Durchgänge über die Zeit bestimmen, sind die Anzahl der Durchgänge und die Startzeitpunkte der Durchgänge. Insbesondere ist es bevorzugt, wie bereits erwähnt, die Startzeitpunkte der Durchgänge gleichmäßig oder zufällig über die Zeit zu verteilen.

Durch ein Verteilen von Durchgängen über eine Mindestzeit wird verhindert, dass z.B. sämtliche Durchläufe in einem quasistatischen Bewegungszustand erfolgen, was sich negativ auf eine statistische Mittelung über die Bewegung auswirken kann.

Es ist bevorzugt, die Bewegungsperiode vor dem Beginn der Bestrahlung zu bestimmen. Dies ist apparativ besonders einfach. Vorzugsweise werden dabei auch Maßnahmen ergriffen, um die Periode der zyklischen Bewegung während der Bestrahlung zumindest ungefähr konstant zu halten. Wird beispielsweise eine Person bestrahlt, so kann sie versuchen, ihre Atembewegung konstant zu halten. Der Erfolg solcher Bemühungen kann durch vorbereitende Maßnahmen, etwa so genanntes "breath coaching", also ein Training, verbessert werden. Zusätzlich kann dem Patienten eine Solltrajektorie beispielsweise über einen Monitor vorgeben werden.

Alternativ oder zusätzlich kann die Periode durch Überwachen der Bewegung während der Bestrahlung vorzugsweise mehrfach oder kontinuierlich bestimmt werden. So können Bestrahlungsparameter, wie etwa die Anzahl oder Startzeitpunkte der Durchgänge, an eine sich während der Bestrahlung ändernde Periode angepasst werden. Vorzugsweise wird die Periode kontinuierlich überwacht und bestimmt, was eine schnelle Anpassung der Bestrahlungsparameter erlaubt.

Beispielsweise kann bei der Überwachung der Atembewegung einer Person auf das Heben und Senken des Brustkorbs abgestellt werden, was messtechnisch leicht realisierbar ist. Es ist auch möglich, die Bewegung des Zielgebiets direkt zu messen, z.B. durch kontinuierliche bzw. in Zeitintervallen aufgenommene Röntgenbilder oder ähnliche Messverfahren, die interne Bewegungen erfassen können.

Es ist grundsätzlich auch vorteilhaft, die zukünftige Bewegung anhand der bestimmten Periode bzw. der beobachteten Bewegung zu prognostizieren; so können Strahlungsparameter auch mit Blick auf die Zukunft angepasst werden.

Um sicherzustellen, dass die Durchgänge der Mehrfachbestrahlung in unterschiedlichen Phasen der zyklischen Bewegung beginnen, kann die zyklische Bewegung, wie oben dargestellt, überwacht werden. Es ist bevorzugt, die Bestrahlung zu stoppen, wenn die Bewegung für mehr als 50% der Bewegungsperiode ruht, denn wenn die Bewegung ruht, wird nicht über verschiedene Bewegungszustände gemittelt. Auch im Vergleich zur Bestrahlungszeit kurze Ruhepausen können sich bereits in einer verstärkten Fehldosierung auswirken. Dabei kann etwa auf eine aktuelle Schätzung beziehungsweise Näherung der Bewegungsperiode vor dem Ruhen der Bewegung abgestellt werden. Zunehmend bevorzugt ist es, die Bestrahlung bereits zu stoppen, wenn die Bewegung für mehr als 30% beziehungsweise 20% oder sogar nur 10% der Bewegungsperiode ruht. Es ist vorteilhaft, wenn sich dabei auch eine Verteilung der Durchgänge über mehrere oder sogar viele Zyklen ergibt.

Ein Desynchronisieren des Ablaufs des Bestrahlens von der zyklischen Bewegung kann auch dadurch erzielt werden, dass der die Rasterpunkte anfahrende Strahl die zyklische Bewegung des Zielgebietes gar nicht "sieht". Bei einer zyklischen Bewegung mit Verweilzeiten beziehungsweise bei langsam durchlaufenen Umkehrpunkten kann die Bestrahlung innerhalb dieser Zeitabschnitte erfolgen. Das sich insgesamt zyklisch bewegende Zielgebiet ist innerhalb dieser Zeitabschnitte quasi unbewegt. Es resultiert eine Reduktion der Wechselwirkung zwischen Mehrfachbestrahlung und der Bewegung und zusätzlich eine Verminderung der Bewegung während der Bestrahlung.

Es ist entsprechend bevorzugt, die zyklische Bewegung zu überwachen und einen Durchgang nur während eines Ruhens der Bewegung zu starten.

Weiter ist es bevorzugt, die zyklische Bewegung zu überwachen und deren Verlauf zu prognostizieren. Es ist dann möglich, einen Durchgang nur innerhalb einer Verweilzeit zu beginnen, wenn die Prognose erwarten lässt, dass der Durchgang während der Verweilzeit zumindest zu 80%, besser vollständig, abgeschlossen sein wird. Dies ist insbesondere bei volumetrischem Anfahren (s.u.) vorteilhaft.

Bei einer bevorzugten Ausführungsform der Erfindung wird die Abfolge des Anfahrens der Rasterpunkte innerhalb des Zielrasters, also der Pfad, von einem der Durchgänge zu einem anderen der Durchgänge verändert. Vorzugsweise wird der Pfad von Durchgang zu Durchgang verändert. Dies ist sowohl für flächige als auch räumliche Zielgebiete bevorzugt sowie sowohl für schichtweises als auch volumetrisches Anfahren. Selbst wenn eine Synchronisation zwischen der Bewegung und dem Starten der Durchläufe vorliegen sollte, resultieren unterschiedliche lokale Fehldosierungen, die sich wieder ausmitteln bzw. kompensieren können.

Kann die zyklische Bewegung beeinflusst werden, was etwa bei der Atembewegung einer Person der Fall sein kann, so kann ein Desynchronisieren auch über eine Beeinflussung der zyklischen Bewegung erzielt werden. Beispielsweise kann etwa die Dauer des Zyklus regelmäßig oder zufällig geändert werden oder die zyklische Bewegung in bestimmten Zeitabschnitten ruhen beziehungsweise zu ausgewählten, etwa gleichmäßig verteilten oder auch zufälligen Zeitpunkten, beginnen.

Vorzugsweise werden während der Bestrahlung über eine Mitteilungseinrichtung Informationen betreffend die zyklische Bewegung angegeben. Diese Informationen können zur Beeinflussung der zyklischen Bewegung, wie oben dargestellt, genutzt werden. Beispielsweise kann eine Person über die Mitteilungseinrichtung in regelmäßigen oder zufällig verteilten Abständen aufgefordert werden, ihre Atemperiode zu verändern. Besonders geeignet ist hier audio-visuelles Feedback.

Die Bestrahlung inkl. aller Durchläufe kann so ausgelegt werden, dass sich für einen bestimmten Bewegungsablauf die Solldosis ergibt. Mit Hilfe der Mitteilungsvorrichtung kann dieser bestimmte Bewegungsablauf etwa auch vorgegeben werden.

Weiter ist es bevorzugt, zumindest einen Teil der Durchgänge mit einer Zeitverzögerung zufälliger Dauer nach dem jeweils vorangehenden Durchgang zu beginnen. Vorzugsweise wird jeder der Durchgänge mit einer Zeitverzögerung zufälliger Dauer gestartet. Auch solche zufälligen Zeitverzögerungen können eine Desynchronisierung bewirken, da die Durchgänge nicht mit identischem zeitlichen Abstand, also periodisch, wiederholt werden. Das Ausbilden einer Synchronisation wird entsprechend gestört. Jeweils exakt die gleichen zufälligen Zeitverzögerungen bzgl. der Bewegung sind unwahrscheinlich. Idealerweise sind die zufälligen Zeitverzögerungen so gewählt, dass die Durchgänge über eine, ggf. variable, Zeit verteilt sind. Beispielhaft kann diese Zeit 50% besser 100% oder sogar 150% der Periodendauer entsprechen, so dass z.B. bei 100% sichergestellt ist, dass zu allen Phasen der Bewegung ein Durchgang stattgefunden hat. Aber auch eine Verteilung über zumindest zwei Zyklen oder eine andere wählbare längere Zeit kann sinnvoll sein.

Das Verfahren kann, wie oben bereits erwähnt, insbesondere auch vorteilhaft mit einem räumlichen Zielgebiet, also einem Zielvolumen, eingesetzt werden. Die Solldosisverteilung ist dann entsprechend eine räumliche Verteilung; auch das Zielraster ist dann räumlich ausgestaltet. Wie oben bereits erwähnt, sind bei räumlichen Zielgebieten Partikelstrahlen besonders vorteilhaft.

Bei einer bevorzugten Ausgestaltung wird ein erstes Teilvolumen des Zielvolumens in mehr Durchgängen bestrahlt als ein zweites Teilvolumen. Vorteilhafterweise werden mehr als zwei Teilvolumina mit jeweils unterschiedlich vielen Durchgängen bestrahlt. Bevorzugt ist es insbesondere bei schichtweisem Anfahren, Schichten mit unterschiedlich vielen Durchgängen zu bestrahlen. Auch das Belegen von einzelnen Rasterpunkten mit jeweils unterschiedlich vielen Durchgängen, etwa von Rasterpunkt zu Rasterpunkt, kann vorteilhaft sein; dabei können die Rasterpunkte auch innerhalb einer Schicht bzw. innerhalb eines Teilvolumens liegen.

So können stärker bewegte Teilvolumina, Schichten oder Rasterpunkte mit einer größeren Anzahl Durchläufe belegt werden als weniger bewegte; bei diesen reicht ggf. bereits ein Durchgang zur Applikation der Solldosis, insb. falls diese sich in Ruhe befinden. Die Bestrahlung kann so insgesamt schneller ausgeführt werden.

Weiter kann es wegen der sog. Vorbestrahlung (s.u.) sinnvoll sein, distale Teilvolumina bzw. Schichten oder Rasterpunkte mit mehr Durchgängen zu belegen als proximale Teilvolumina. So kann etwa eine Unterteilung sinnvoll sein, bei der Teilvolumina/Schichten/Rasterpunkte sukzessive von distal nach proximal mit weniger Durchgängen belegt werden. Mit anderen Worten kann man auch von einer asymmetrischen Anzahl von Durchgängen sprechen.

Ein Zielvolumen, beziehungsweise das zugehörige räumliche Zielraster, werden oft schichtweise bestrahlt. Vorteilhaft ist es hier, den Beginn des Bestrahlens für zumindest einen Teil der Schichten innerhalb eines Durchgangs nach einer zufälligen Zeitverzögerung durchzuführen. Noch besser ist es, den Beginn des Bestrahlens für sämtliche Schichten innerhalb eines Durchgangs nach einer zufälligen Zeitverzögerung durchzuführen; idealerweise wird so in sämtlichen Durchgängen verfahren. So wird eine ungünstige Synchronisation zwischen den Durchgängen in aufeinander folgenden Schichten des Volumens erschwert.

Die zufälligen Zeitverzögerungen zwischen dem Beginn des Bestrahlens einzelner Schichten unterstützen auch eine Desynchronisierung des Ablaufs des Bestrahlens insgesamt von der zyklischen Bewegung. Zum einen entspricht jede Verzögerung einer Phasendifferenz bezüglich der zyklischen Bewegung, so dass auch eine Kompensation des Einflusses der Schichten auf die deponierte Dosis über die Phasen der Bewegung unterstützt wird. Zusätzlich weisen die Durchgänge unterschiedliche Dauern zufälliger Länge auf, was, etwa wie bei den zufälligen Zeitverzögerungen zwischen den Durchgängen, desynchronisierend wirkt. Jeweils exakt die gleichen zufälligen Zeitverzögerungen bei Bewegung sind äußerst unwahrscheinlich.

Gegeben ein Zielvolumen und ein räumliches Zielraster, welches schichtweise abgetastet wird, so ist es auch bevorzugt, den Pfad in einer Schicht von einem der Durchgänge zu einem anderen der Durchgänge zu verändern; Bevorzugter ist es, den Pfad in einer Schicht in jedem Durchgang zu ändern. Dadurch wird das Entstehen einer lokalen Fehldosierung erschwert, da Rasterpunkte in einer Schicht zu unterschiedlichen Zeitpunkten bezogen auf den Startzeitpunkt des jeweiligen Durchgangs angefahren werden.

Es ist auch bevorzugt, den Pfad von einer ersten der Schichten zu einer zweiten der Schichten zu ändern. Bevorzugter wird der Pfad von Schicht zu Schicht, idealerweise bei jedem Wechsel der Schicht, geändert. Selbst wenn eine Synchronisation zwischen Bewegung und Durchläufen pro Schicht vorliegt, ergeben sich so jeweils andere Wechselwirkungsmuster, die zu einer Mittelung führen.

Wird das Zielvolumen nicht in Schichten abgetastet, sondern entlang eines durch ein Volumen verlaufenden Pfades (volumetrisches rescanning bzw. depth scanning), so ist es ebenfalls bevorzugt, den Pfad von Durchgang zu Durchgang zu verändern.

Insbesondere bei diesem volumetrischen rescanning ist es bevorzugt, unterschiedliche Teilvolumina mit unterschiedlich vielen Durchgängen zu bestrahlen.

Eine erfindungsgemäße Desynchronisierung kann auch durch eine Modulation der Strahlgeschwindigkeit erzielt werden, welche vorzugsweise mit einer Modulation der Intensität des Strahls realisiert wird. Je höher die Intensität des Strahls, umso höher kann die Strahlgeschwindigkeit gewählt werden. Variiert die Strahlgeschwindigkeit, so werden Verzögerungen und Beschleunigungen in das Bestrahlen der Schichten beziehungsweise in die Abfolge der Durchgänge eingebaut, welche sich grundsätzlich genauso auswirken, wie etwa eine Variation der Startzeitpunkte von Schichten beziehungsweise Durchgängen. Eine Veränderung der Bewegungsparameter synchron zur Intensitätsänderung ist extrem unwahrscheinlich, was sich insb. wiederum in einer Mittelung über Fehldosierungen auswirkt.

Auch kann die Periode wie oben dargestellt bestimmt und ggf. auch prognostiziert werden, um durch Anpassen der Strahlintensität die Abtastgeschwindigkeit so einzustellen, dass die Durchgänge über eine der oben genannten Zeiten verteilt sind.

Die Intensität des Strahls kann während einer Extraktion aus einem Beschleuniger, etwa einem Synchrotron oder einem Zyklotron, verändert werden. Vorzugsweise wird diese Intensitätsveränderung mit den anderen Bestrahlungsparametern abgestimmt; So kann etwa darauf abgezielt werden, nicht von der für die Rasterpunkte zu deponierenden Dosis abzuweichen. Es bietet sich aber auch eine zufällige Modulation der Strahlintensität zur Beeinflussung der Geschwindigkeit an, mit der die Rasterpunkte angefahren werden.

In der Tiefe kann die Abtastgeschwindigkeit (depth scanning bzw. volumetrisches scanning) etwa durch ein Absorberpaar oder ein Modulatorrad eingestellt werden.

Auch betreffend die Modulation der Strahlintensität kann eine Anwendung über mehrere Zyklen erfolgen, insbesondere können die Durchgänge über mehrere Zyklen verteilt sein.

Grundsätzlich kann ein Zielvolumen auch sowohl schichtweise als auch volumetrisch angefahren werden, etwa nacheinander, alternierend oder auch kombiniert. Beispielsweise kann das Zielvolumen zunächst mehrfach volumetrisch bestrahlt werden und anschließend mehrfach schichtweise.

Bei sämtlichen Ausgestaltungen der Erfindung kann die Bestrahlung über mehrere Sitzungen verteilt sein (auch sog. fraktionierte Bestrahlung); dabei soll sich die insgesamt gewünschte Dosisverteilung nicht innerhalb einer Sitzung, sondern nach Abschluss aller Sitzungen ergeben. Es ergibt sich eine Mittelung von Fehldosierungen über mehrere Sitzungen. Insbesondere kann die Ausgestaltung der Mehrfachbestrahlung auch von Sitzung zu Sitzung geändert werden; ein Durchgang einer Mehrfachbestrahlung entspricht im Extremfall einer Einfachbestrahlung während einer Sitzung. Besonders bevorzugt ist die Anwendung des Verfahrens auf eine einzelne, insbesondere auf jede einzelne, Sitzung, bei der während dieser einzelnen Sitzung eine Mehrfachbestrahlung durchgeführt wird, d.h. wenigstens ein Teil der Punkte wird mehrfach angefahren.

Die Erfindung betrifft weiter eine Bestrahlungsvorrichtung zur Deposition einer Solldosisverteilung in einem zyklisch bewegten Zielgebiet durch Mehrfachbestrahlung mit einer Strahlerzeugungsvorrichtung und einer Steuerungsvorrichtung, welche Stahlerzeugungsvorrichtung und Steuerungsvorrichtung dazu ausgelegt sind, mit einem Strahl Rasterpunkte eines Zielrasters in einer Anzahl von zumindest zwei Durchgängen anzufahren, wobei in jedem der Durchgänge Rasterpunkte aufeinanderfolgend angefahren werden. Die Bestrahlungsvorrichtung ist dadurch gekennzeichnet, dass die Steuerungsvorrichtung dazu ausgelegt ist, den Ablauf der Bestrahlung und die zyklische Bewegung des Zielgebiets zu desynchronisieren durch Veränderung zumindest einer der folgenden Steuerungsparameter: Abtastgeschwindigkeit des Strahls; Zeitliche Verteilung der Durchgänge, wobei die Durchgänge zufällig verteilt werden oder wobei Pausenzeiten zwischen einzelnen Durchgängen variiert werden; zeitliche Verteilung des Bestrahlens von Schichten; zeitliche Verteilung des Bestrahlens von bestimmten Volumina innerhalb der einzelnen Durchgänge; Anfangsphase der zyklischen Bewegung, wobei über eine Mitteilungseinrichtung einer Person Informationen betreffend die zyklische Bewegung angegeben werden; Zyklusdauer der zyklischen Bewegung, wobei die Dauer des Zyklus zufällig geändert wird; Pfad des Strahls im Zielgebiet und dazu ausgelegt ist, die Bestrahlung des Zielgebiets auf bis zu 30 Durchgänge aufzuteilen, so dass lokale Fehldosierungen klein gehalten werden.

Als Strahlerzeugungsvorrichtung kommt etwa ein Beschleuniger, insbesondere ein Synchrotron oder ein Zyklotron in Betracht. Die Steuervorrichtung ist dazu ausgelegt den Ablauf der Bestrahlung zu bestimmen und kann auch als Festlegungseinheit fungieren. Sie kann etwa einen entsprechend programmierten Rechner, mit dem Rechner steuerbare Mittel zur räumlichen Beeinflussung des Strahls und Mittel zur Messung der Strahlintensität umfassen. Der Rechner kann etwa die Festlegungseinheit umfassen, etwa in der Art, dass der Programmierung des Rechners die maximal tolerierte Abweichung von der Solldosisverteilung zu Grunde liegt, insbesondere explizit hinterlegt bzw. codiert ist.

Die Bestrahlungsvorrichtung ist auch zur Ausführung des erfindungsgemäßen Verfahrens in allen bevorzugten Ausgestaltungen ausgelegt.

Weiter bezieht sich die Erfindung auch auf ein Verfahren zur Bestimmung von Steuerungsparametern für obige Bestrahlungsvorrichtung zur Durchführung des Verfahrens zur Deposition einer Solldosisverteilung in einem zyklisch bewegten Zielgebiet, auch in allen bevorzugten Ausgestaltungen, durch Mehrfachbestrahlung mit einem Rasterpunkte eines Zielrasters anfahrenden Strahl in einer Anzahl von zumindest zwei Durchgängen, bei dem in jedem der Durchgänge Rasterpunkte aufeinanderfolgend angefahren werden. Dieses Parameterbestimmungsverfahren ist gekennzeichnet durch die Schritte: Bestimmen der Vorgehensweise für die Desynchronisierung des Ablaufs der Bestrahlung und der zyklischen Bewegung des Zielgebiets, wobei zumindest einer der folgenden Steuerungsparameter: Abtastgeschwindigkeit des Strahls; zeitliche Verteilung der Durchgänge, wobei die Durchgänge zufällig verteilt werden oder wobei Pausenzeiten zwischen einzelnen Durchgängen variiert werden; zeitliche Verteilung des Bestrahlens von Schichten; zeitliche Verteilung des Bestrahlens von bestimmten Volumina innerhalb der einzelnen Durchgänge; Zyklusdauer der zyklischen Bewegung, wobei die Dauer des Zyklus zufällig geändert wird; Pfad des Strahls im Zielgebiet zur Desynchronisation des Ablaufs der Bestrahlung von der zyklischen Bewegung des Zielgebiets verändert wird, und Aufteilung der Bestrahlung des Zielgebiets auf bis zu 30 Durchgänge, so dass lokale Fehldosierungen klein gehalten werden. Ein "Bestimmen" umfasst insbesondere auch "Festlegen", so kann etwa die maximal tolerierte Abweichung von der Solldosisverteilung festgelegt werden.

Es ist für das Verständnis der Erfindung hilfreich zu wissen, dass die maximal tolerierbare lokale Abweichung von der Solldosisverteilung etwa empirisch bestimmt werden kann; Sie ergibt sich insbesondere aus den Eigenschaften des zu bestrahlenden Ziels, der Solldosisverteilung und des angestrebten Bestrahlungserfolges. Die Bestimmung der Vorgehensweise für die Desynchronisierung als einen die Bestrahlung charakterisierenden Parameter entspricht im einfachsten Fall der Auswahl zwischen den angegebenen desynchronisierend wirkenden Maßnahmen. Eine Aufteilung der Bestrahlung in hinreichend viele Durchgänge kann etwa empirisch erfolgen oder anhand von Modellrechnungen.

Die vorangehende und die folgende Beschreibung der einzelnen Merkmale bezieht sich sowohl auf die Vorrichtungskategorie als auch auf die Verfahrenskategorie, ohne dass dies im Einzelnen in jedem Fall explizit erwähnt ist; die dabei offenbarten Einzelmerkmale können auch in anderen als den gezeigten Kombinationen erfindungswesentlich sein.

Im Folgenden soll die Erfindung auch anhand von Ausführungsbeispielen näher erläutert werden.
Fig. 1 zeigt eine schematische Darstellung einer erfindungsgemäßen Bestrahlungsvorrichtung zur Durchführung der erfindungsgemäßen Verfahren.
Fig. 2 zeigt ein Ablaufschema des erfindungsgemäßen Verfahrens zur Deposition einer Solldosisverteilung.
Fig. 3 zeigt eine schematische Darstellung eines Pfads eines mehrere Rasterpunkte anfahrenden Strahls.
Fig. 4 zeigt ein zweites Ablaufschema des erfindungsgemäßen Verfahrens zur Deposition einer Solldosisverteilung.
Fig. 5 zeigt eine Auswahl desynchronisierender Maßnahmen
Fig.6 zeigt ein Ablaufschema des erfindungsgemäßen Verfahrens zur Bestimmung von Steuerungsparametern.

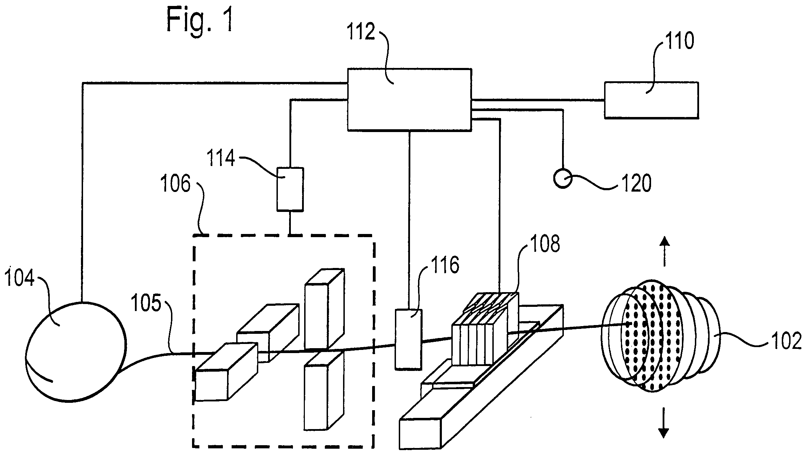

Die Bestrahlungsvorrichtung ist dazu ausgelegt, ein Zielvolumen 102 zu bestrahlen. Bei dem Zielvolumen 102 handelt es sich etwa um einen nahe bzw. in der Lunge einer Person gelegenen Tumor. Alternativ kann es sich auch um ein Phantom, etwa basierend auf Wasser oder Plexiglas, oder ein sonstiges Material handeln. Das Zielvolumen bewegt sich zyklisch etwa auf und ab, was in Fig. 1 durch die Pfeile über und unter dem Zielvolumen 102 angedeutet ist.

Die Bestrahlungsvorrichtung weist ein Synchrotron, ein Zyklotron oder einen sonstigen Beschleuniger 104 zur Bereitstellung eines Partikelstrahls 105, bspw. bestehend aus Protonen oder ¹²C Kernen, auf. Typischerweise hat so ein Strahl eine Ausdehnung von einem oder mehreren Millimetern, etwa 6 mm oder 10 mm. In dem Zielvolumen 102 sind Schichten angedeutet, welche der Tiefe des Bragg-Peaks für eine bestimmte Teilchenenergie entsprechen (isoenergetische Schichten).

Mit dem Partikelstrahl 105 fährt die Bestrahlungsvorrichtung schematisch als schwarze Punkte in dem Zielvolumen 102 dargestellte Rasterpunkte an. Es ist, wegen der einfacheren Darstellung, schematisch ein schichtweises Anfahren von Rasterpunkten gezeigt; alternativ können Rasterpunkte natürlich auch volumetrisch angefahren werden (nicht dargestellt).

Eine laterale Beeinflussung des Partikelstrahls 105 kann mittels Scanmagneten 106 erfolgen. Hier handelt es sich um Dipolmagnete 106. Für eine longitudinale (entlang der Strahlrichtung) Beeinflussung weist die Bestrahlungsvorrichtung eine passive Energievariationseinrichtung etwa in Form eines Keilsystems 108 zur Energiemodulation auf. Das Keilsystem 108 weist Keile etwa aus Kunststoff auf, die über einen linearen Motor (nicht gezeigt) bewegt werden können. Das Keilsystem 108 wird bevorzugt bei volumetrischen Scans verwendet. Wird schichtweise gescannt so wird die Energie bevorzugt über den Beschleuniger oder eine vor den Scanmagneten 105 installierte Energiemodulationseinheit geändert.

Weiter weist die Bestrahlungsvorrichtung eine Erfassungseinrichtung 110, eine Kontrolleinrichtung 112, einen Rasterscan 114 und einen Teilchenzähler 116 auf. Die Erfassungseinrichtung 110 ist dazu ausgelegt, eine Bewegung des Zielvolumens 102 zu erfassen; dazu weist sie ein Detektorsystem zur Aufnahme der Bewegung des Zielgebiets selbst oder ersatzweise einer anderen damit korrelierten Bewegung auf, beispielsweise eine Kamera zur Detektion der Brustoberflächenbewegung.

Die Erfassungseinrichtung 110 führt der Kontrolleinrichtung 112 die gewonnenen Daten über die Bewegung des Zielvolumens 102 zu. Der Teilchenzähler 116 bestimmt die Anzahl der Teilchen in dem Partikelstrahl 105 und führt das Ergebnis ebenfalls der Kontrolleinrichtung 112 zu. Bei dem Teilchenzähler 116 kann es sich um eine Ionisationskammer handeln. Die auch als Steuerung fungierende Kontrolleinrichtung 112 ist dazu ausgelegt, den Beschleuniger 104, die Scanmagneten 106 und das Keilsystem 108 zu steuern. Dazu ermittelt die Kontrolleinrichtung 112 die entsprechenden Steuerungsparameter unter Berücksichtigung der von der Erfassungseinrichtung 110 und von dem Teilchenzähler 116 empfangenen Daten.

Über eine von der Kontrolleinrichtung 112 gesteuerte Mitteilungseinrichtung 120 können etwa einer zu bestrahlenden Person Informationen mitgeteilt werden. Die Mitteilungseinrichtung 120 kann Töne und Lichtsignale abgeben, sie kann etwas einen LCD-Bildschirm und eine Sprachausgabe umfassen.

Die Bestrahlungsvorrichtung wird erfindungsgemäß dazu genutzt, in dem Zielvolumen 102 eine bestimmte Dosis mit bestimmter Verteilung zu deponieren. Dazu wird die Bestrahlung in mehrere Durchgänge aufgeteilt, wobei in jedem der Durchgänge die Rasterpunkte teilweise oder aber auch alle nacheinander angefahren werden. Die Anzahl der Durchgänge wird so gewählt, dass eine bestimmte Abweichung von der Solldosisverteilung, etwa von 1,5%, nicht überschritten wird.

Um dabei Fehldosierungen über die Durchgänge zumindest teilweise zu kompensieren werden erfindungsgemäß einer oder mehrere für die Bestrahlung relevante Parameter während der Bestrahlung verändert.

Dies betrifft beispielsweise die Strahlintensität, und damit üblicherweise zusammenhängend, die Geschwindigkeit mit der die Rasterpunkte angefahren werden, die zeitliche Verteilung der Durchgänge, etwa bezogen auf den Startzeitpunkt der jeweiligen Durchgänge, die zeitliche Verteilung des Bestrahlens von Schichten bzw. von bestimmten Volumina innerhalb der einzelnen Durchgänge, etwa bezogen auf den Beginn des Bestrahlens einer Schicht bzw. eines Volumens - etwa durch modulierte Beschleunigerextraktion oder durch passive Energiemodulation -, die Wahl der Anfangsphase und/oder der Zyklusdauer beziehungsweise einer dafür angenommenen Dauer der Bewegung, den Pfad des Strahls im Zielvolumen 102, insbesondere innerhalb von Schichten bzw. Volumina innerhalb des Zielvolumens 102.

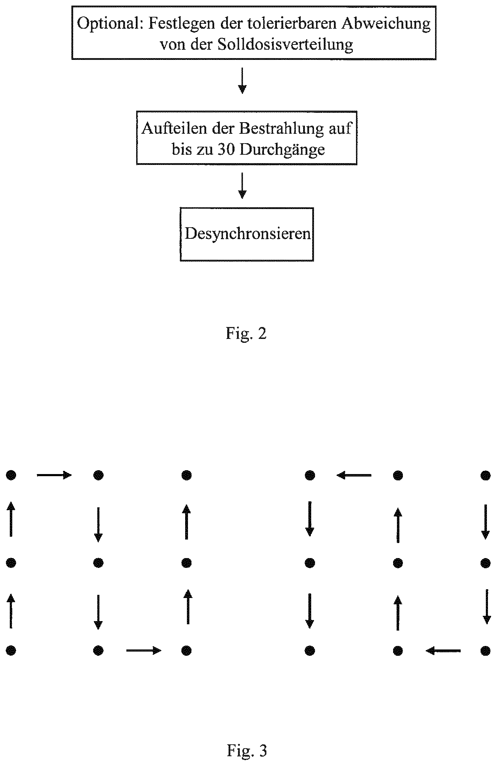

Die oben dargestellten Grundzüge des Verfahrens sind in dem in Fig. 2 dargestellten Ablaufschema aufgenommen.

Steuerungsparameter können von der zyklischen Bewegung abhängen. Hierzu wird eine Eigenschaft der zyklischen Bewegung in ein Signal umgesetzt und der Kontrolleinrichtung 112 zur Steuerung der Mehrfachbestrahlung zugeführt.

Handelt es sich bei dem zyklisch bewegten Ziel bspw. um einen durch die Atmung einer Person bewegten Tumor, so kann etwa über die Erfassungseinrichtung 110 das Heben und Senken des Brustkorbs der Person erfasst werden, die mittlere Atemperiode und ggf. auch die mittlere Amplitude bestimmt und der Kontrolleinrichtung 112 zur Steuerung der Mehrfachbestrahlung zugeführt werden. Es ist auch möglich, die Bewegung des Zielgebiets direkt zu messen, z.B. durch kontinuierliche bzw. in Zeitintervallen aufgenommene Röntgenbilder oder ähnliche Messverfahren, die interne Bewegungen erfassen können.

Es können beispielsweise einer oder mehrere der folgenden Parameter der Mehrfachbestrahlung an die zyklische Bewegung angepasst werden, um die gewünschte Abweichung von der Solldosisverteilung nicht zu überschreiten:
- die Strahlintensität während eines Durchgangs;
- die Geschwindigkeit mit der der Strahl die Rasterpunkte innerhalb eines Durchgangs von Rasterpunkte zu Rasterpunkt wechselt;
- die Startzeitpunkte der einzelnen Durchgänge.

Die konkrete Maßnahme zur Desynchronisierung hängt u.a. von dem zu bestrahlenden Ziel ab und kann etwa empirisch oder anhand von Modellrechnungen bestimmt werden.

Die Startzeitpunkte der einzelnen Durchgänge können dabei etwa über einen Abschnitt der zyklischen Bewegung, über einen Zyklus oder über eine Zeit, die größer ist als eine Zyklusdauer, verteilt sein. Die Startzeitpunkte können dabei etwa regelmäßig aufeinander folgend oder auch zufällig verteilt sein.

Das eine Eigenschaft der zyklischen Bewegung ausdrückende Signal kann etwa im Rahmen einer Planungsphase vor der eigentlichen Bestrahlung bestimmt werden. Es wird im Vorfeld also eine typische beziehungsweise objektspezifische, etwa eine personenspezifische, Eigenschaft der zyklischen Bewegung bestimmt. Das der Kontrolleinrichtung 112 zuzuführende Signal kann auch, gegebenenfalls zusätzlich, während der Bestrahlung mehrfach beziehungsweise sogar kontinuierlich bestimmt werden (online).

Wird eine Eigenschaft der zyklischen Bewegung vor der eigentlichen Bestrahlung bestimmt, kann etwa auf eine mittlere oder auf eine typische Bewegungsperiode abgestellt werden. Ist die Bewegungsperiode bekannt, so können die Bestrahlungsparameter entsprechend angepasst werden, so können etwa die Startzeitpunkte der Durchgänge gleichmäßig oder zufällig verteilt werden; oder die Abtastgeschwindigkeit so angepasst werden, dass die Durchgänge über die unten noch einmal explizit genannten Zeiträume verteilt sind. Damit sich eine Anpassung der Bestrahlungsparameter während der Bestrahlung erübrigt, ist es sinnvoll, wenn die Bewegungsperiode über die Bestrahlung konstant bleibt. Beispielsweise kann eine Person im Vorfeld so trainiert werden, dass Ihre Atmungsperiode vergleichsweise konstant bleibt (breath coaching).

Die Bewegung kann aber auch mit der in Fig.1 gezeigten Erfassungseinrichtung 110 während der Bestrahlung erfasst werden und so mehrfach oder auch kontinuierlich überprüft werden.

So kann die aktuelle Bewegungsperiode beziehungsweise auch die Phase der Bewegung bestimmt werden, welche dann an die Kontrolleinrichtung 112 übermittelt wird. Eine Bestimmung der Phase ist beispielsweise bei einer Person etwa über das Heben und Senken des Brustkorbs leicht zu realisieren. Mit Hilfe der Erfassung der Bewegungsperiode bzw. Phase der Bewegung können die einzelnen Durchgänge gezielt in unterschiedlichen Phasen der Bewegung gestartet werden.

Mit Hilfe der Mitteilungseinrichtung 120 kann etwa auch einer zu bestrahlenden Person mit einem Instruktionssignal vorgegeben werden, dass sie etwa ihre Atemperiode oder Phase zu bestimmten Zeitpunkten oder auch zu zufälligen Zeitpunkten während der Bestrahlung zu ändern habe. Hier wird die Person in gleichmäßig über die Bestrahlungszeit verteilten Zeitpunkten mittels der Mitteilungseinrichtung 120 aufgefordert, die Atembewegung zu ändern. Die Signale können akustischer und/oder optischer Natur sein.

Es soll noch einmal betont werden, dass zusätzlich oder alternativ zu einer von der zyklischen Bewegung abhängigen Ausgestaltung der Bestrahlung auch eine Eigenschaft der Bestrahlung während der Bestrahlung geändert werden kann. Auch dies kann zu regelmäßig oder zufällig verteilten Zeitpunkten geschehen.

So können etwa die oben genannten Parameter während einer Sitzung geändert werden. Weiter können aber etwa auch Pausenzeiten zwischen einzelnen Durchgängen oder der Pfad des Strahls während einer Sitzung verändert werden. In diesem Beispiel wird einer oder werden mehrere der Parameter von Durchgang zu Durchgang verändert. Alternativ oder zusätzlich werden die Parameter von Sitzung zu Sitzung verändert.

Bei einer erfindungsgemäßen Ausgestaltung des Bestrahlungsverfahrens wird die Bestrahlung in mehrere Durchgänge aufgeteilt und die Durchgänge derart über die zyklische Bewegung verteilt, dass die Durchgänge in unterschiedlichen Phasen der zyklischen Bewegung appliziert werden. Dabei können auch hier die Startzeitpunkte gleichmäßig aufeinander folgend gewählt sein oder auch zufällige Zeitverzögerungen zwischen den Durchgängen eingebracht werden. Die Durchgänge können etwa über eine typische oder mittlere Bewegungsperiode oder eine Zeit länger als eine Bewegungsperiode verteilt werden, bspw. 150% der Bewegungsperiode. Alternativ kann auch eine Gesamtbestrahlungsdauer von zwei Bewegungsperioden oder drei Bewegungsperioden oder eine andere länger als einen Zyklus währende Dauer gewählt werden.

Es können etwa 5, besser 15 bis 20 oder bis 30 Durchgänge gewählt werden. Typischerweise ist das Zielvolumen in 50 Schichten unterteilt. Es dauert typischerweise 100ms bis zu 1s, um eine Schicht abzufahren. Wird ein Volumen angefahren, liegt die Anfahrdauer typischerweise im Bereich von Sekunden.

Die Anzahl der Durchgänge kann für verschiedene Isoenergieschichten oder sonstige Teilvolumina auch unterschiedlich gewählt werden. So bietet es sich an, proximale Schichten seltener anzufahren als distale, da beim Anfahren der distalen Schichten proximale Schichten auch bestrahlt werden (man spricht hier auch von "Vorbestrahlen" der proximalen Schichten).

Bewegt sich ein Teil des Zielgebiets schneller als ein anderes, so können entsprechende Teilvolumina mit mehr Durchgängen belegt werden als langsamer bewegte Teilvolumina.

Weiter kann die Bestrahlung auch gestoppt werden, wenn über die Erfassungseinrichtung 110 ein Ruhen der zyklischen Bewegung festgestellt wird. Ruht die Bewegung für mehr als beispielsweise 10% der bestimmten Bewegungsperiode, so wird die Bestrahlung unterbrochen. Zusätzlich kann die Periode auch prognostiziert werden; stimmen gemessene und prognostizierte Periode nicht ausreichend überein, etwa bei einer Abweichung von mehr als 10%, ist es ebenfalls vorteilhaft, die Bestrahlung zu unterbrechen.

Alternativ kann ein Ruhen der zyklischen Bewegung auch dazu genutzt werden, das Zielvolumen 102 innerhalb einer solchen Ruhephase zu bestrahlen. Dazu wird ebenfalls über die Erfassungseinrichtung 110 die Bewegung verfolgt und in einem Rechner der Erfassungseinrichtung 110 oder der Kontrolleinrichtung 112 der weitere Fortgang der Bewegung prognostiziert (basierend auf einer Auswertung der vorangegangenen Zyklen; etwa durch Extrapolation). Ergibt die Prognose, dass ein Durchgang der Bestrahlung innerhalb der Ruhephase durchgeführt werden kann, so wird dieser gestartet. Betreffend diesen Durchgang wird ein nahezu unbewegtes Zielvolumen 102 bestrahlt.

Der das Zielvolumen 102 bestrahlende Strahl kann einen dreidimensionalen (volumetrischen) Pfad aufweisen oder das Zielvolumen 102 schichtweise abfahren. In beiden Fällen kann der Pfad von Durchgang zu Durchgang verändert werden. Wird schichtweise bestrahlt, so kann die Abfolge der Rasterpunkte in einer der Schichten oder in allen Schichten von Durchgang zu Durchgang verändert werden. Fig. 3 zeigt schematisch je einen Pfad in der gleichen (isoenergetischen) Schicht in zwei unterschiedlichen Durchgängen. Die gezeigten Pfeile zeigen dabei die Reihenfolge des Anfahrens der Rasterpunkte an. In der linken Abbildung verläuft der Pfad von links unten nach rechts oben und in der rechten Abbildung von rechts oben nach links unten.

Wird das Zielvolumen 102 schichtweise abgetastet, so können die Startzeitpunkte für die Bestrahlung der einzelnen Schichten zufällig verteilt gewählt werden beziehungsweise Zeitverzögerungen zufälliger Dauer zwischen die Bestrahlungen der einzelnen Schichten eingeführt werden. Insbesondere können die Zeitverzögerungen so gewählt werden, dass sich insgesamt eine der oben genannten Dauern für eine Sitzung der Mehrfachbestrahlung ergibt. Zusätzlich kann auch von Schicht zu Schicht, auch innerhalb desselben Durchgangs, ein anderer Pfad gewählt werden.

Unabhängig davon, ob das Zielvolumen 102 in Schichten oder volumetrisch abgetastet wird, kann die Abtastgeschwindigkeit mit einer Modulation der Strahlintensität moduliert werden. Dazu kann die Intensität des Strahls während der Extraktion aus dem Beschleuniger definiert, das heißt auf die weiteren Bestrahlungsparameter - inklusive der pro Rasterpunkte zu deponierenden Teilchenzahl - abgestimmt, verändert werden. Die Intensitätsmodulation kann beispielsweise einen zufälligen Verlauf aufweisen. Hier wird die Intensität des Strahls so eingestellt, dass sich die Bestrahlung über die oben erwähnten Zeitintervalle erstreckt.

Die Geschwindigkeit der Tiefenmodulation (depth scanning bei volumetrischem Rescanning) kann etwa über das keilförmige Absorberpaar 108 oder über ein Modulatorrad (nicht gezeigt) mit einem bestimmten Verlauf oder mit einem zufälligen Verlauf verändert werden.

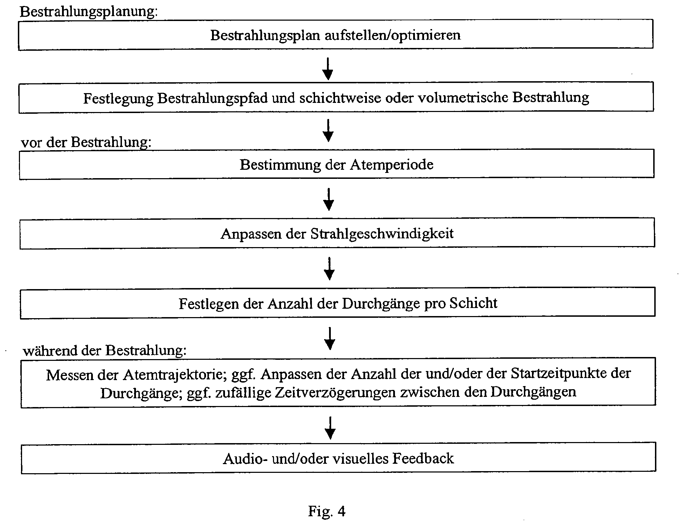

Fig. 4 zeigt einen exemplarischen Ablauf des erfindungsgemäßen Bestrahlungsverfahrens für die Bestrahlung einer Person, bei der die zyklische Bewegung des Ziels durch die Atmung der Person verursacht wird. Hier wird im Vorfeld eine Bestrahlungsplanung durchgeführt, umfassend die Aufstellung beziehungsweise Optimierung des Bestrahlungsplans, die Festlegung, ob schichtweise oder volumetrisch bestrahlt werden soll und die entsprechende Festlegung des Bestrahlungspfads. Es kann auch zunächst ein Bestrahlungspfad festgelegt werden und anschließend entsprechend eingestellt werden, ob schichtweise oder volumetrisch bestrahlt wird. Im Rahmen der Bestrahlungsplanung können auch schon andere die Bestrahlung bestimmende Parameter festgelegt werden, etwa betreffend die, gegebenenfalls zufällig zu startenden, Durchgänge pro Schicht, die Zeitverzögerung zwischen Durchgängen, oder ob ein Feedback an die zu bestrahlende Person durchgeführt wird. Vor der beziehungsweise vor jeder Bestrahlung wird die Atemperiode bestimmt, die Strahlgeschwindigkeit angepasst, also die Geschwindigkeit mit der die Rasterpunkte angefahren werden, und die Anzahl der Durchgänge pro Schicht festgelegt. Während der Bestrahlung wird die Atemtrajektorie gemessen und gegebenenfalls zur Anpassung der Anzahl der Durchgänge beziehungsweise der Startzeitpunkte der Durchgänge verwendet. Gegebenenfalls können die Zeitverzögerungen zwischen den Durchgängen auch zufällig gewählt werden. Die zu bestrahlende Person erhält Audio- und/oder visuelles Feedback betreffend die gemessene Trajektorie und/oder betreffend die Soll-Trajektorie.

Das erfindungsgemäße Bestrahlungsverfahren kann auch verteilt über mehrere Sitzungen (sog. Fraktionen) durchgeführt werden. Die Solldosisverteilung bezieht sich dann auf die insgesamt in sämtlichen Sitzungen zu applizierende Strahlung. Die Mittelung über die Fraktionen kann die Desynchronisierung unterstützen. Der Ablauf der Bestrahlung kann insbesondere auch von Sitzung zu Sitzung unterschiedlich gewählt werden.

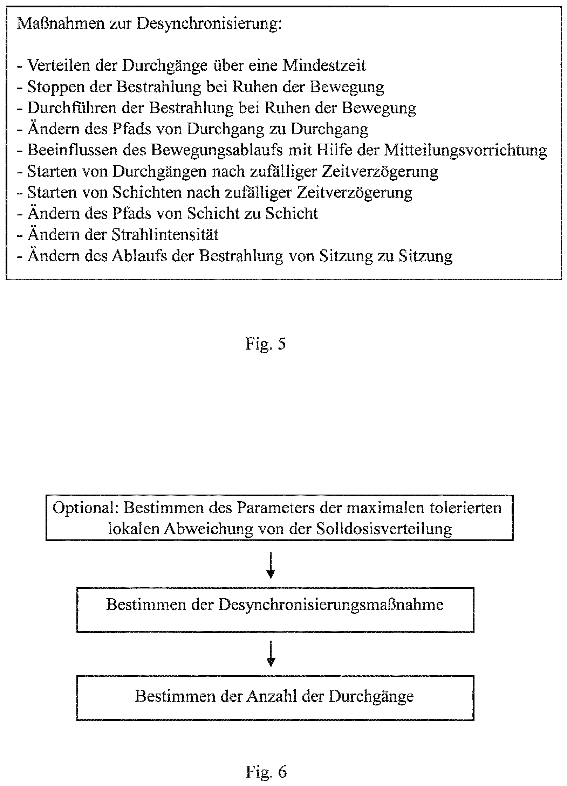

Fig. 5 zeigt eine Auswahl bereits genannter desynchronisierender Maßnahmen zur Übersicht.

Fig. 6 zeigt ein Ablaufschema des Verfahrens zur Bestimmung von Steuerungsparametern für die Bestrahlungsvorrichtung. Es umfasst die Schritte: Bestimmen der Vorgehensweise für die Desynchronisierung des Ablaufs der Bestrahlung und der zyklischen Bewegung des Zielgebiets (102) und Bestimmen der Aufteilung der Bestrahlung des Zielgebiets auf bis zu 30 Durchgänge, so dass lokale Dosisabweichungen klein gehalten werden. Wie oben bereits erwähnt bietet es sich an, diese Parameter empirisch oder anhand von Modellrechnungen zu bestimmen.

## Patentansprüche

1. Verfahren zur Deposition einer Solldosisverteilung in einem zyklisch bewegten Zielgebiet (102), welches nicht in einem Mensch oder Tier angeordnet ist, durch Mehrfachbestrahlung mit einem Rasterpunkte eines Zielrasters anfahrenden Strahl (105) in eine Anzahl von zumindest zwei Durchgängen, bei dem in jedem der Durchgänge Rasterpunkte aufeinanderfolgend angefahren werden, **gekennzeichnet durch** die Schritte:
Desynchronisieren des Ablaufs der Bestrahlung und der zyklischen Bewegung des Zielgebiets (102) durch Veränderung zumindest einer der folgenden Steuerungsparameter:
- Abtastgeschwindigkeit des Strahls,
- zeitliche Verteilung der Durchgänge, wobei die Durchgänge zufällig verteilt werden oder wobei Pausenzeiten zwischen einzelnen Durchgängen variiert werden,
- zeitliche Verteilung des Bestrahlens von Schichten,
- zeitliche Verteilung des Bestrahlens von bestimmten Volumina innerhalb der einzelnen Durchgänge,
- Zyklusdauer der zyklischen Bewegung, wobei die Dauer des Zyklus zufällig geändert wird,
- Pfad des Strahls im Zielgebiet, und
Aufteilen der Bestrahlung des Zielgebiets auf bis zu 30 Durchgänge, so dass lokale Fehldosierungen klein gehalten werden.

2. Verfahren nach Anspruch 1,
bei dem zumindest ein Teil der Rasterpunkte in unterschiedlichen Durchgängen gezielt in unterschiedlichen Phasen der zyklischen Bewegung angefahren wird oder bei dem zumindest ein Teil der Rasterpunkte in unterschiedlichen Durchgängen gezielt in unterschiedlichen Phasen der zyklischen Bewegung angefahren wird und bei dem die zyklische Bewegung überwacht wird.

3. Verfahren nach einem der vorangehenden Ansprüche,
bei dem die Bewegungsperiode bestimmt wird und zumindest ein Teil der Durchgänge über eine Zeit verteilt ist, die zumindest 50% einer Bewegungsperiode entspricht oder
bei dem die Bewegungsperiode bestimmt wird und zumindest ein Teil der Durchgänge über eine Zeit verteilt ist, die zumindest 50% einer Bewegungsperiode entspricht und bei dem die Bewegungsperiode vor der Bestrahlung bestimmt wird oder
bei dem die Bewegungsperiode bestimmt wird und zumindest ein Teil der Durchgänge über eine Zeit verteilt ist, die zumindest 50% einer Bewegungsperiode entspricht und bei dem die Bewegungsperiode durch Überwachen der Bewegung während der Bestrahlung mehrfach bestimmt wird oder
bei dem die Bewegungsperiode bestimmt wird und zumindest ein Teil der Durchgänge über eine Zeit verteilt ist, die zumindest 50% einer Bewegungsperiode entspricht und bei dem die Bewegungsperiode vor der Bestrahlung bestimmt wird und bei dem die Bewegungsperiode durch Überwachen der Bewegung während der Bestrahlung mehrfach bestimmt wird und/oder
bei dem die Bewegung überwacht wird und die Bestrahlung gestoppt wird, wenn die Bewegung für mehr als 50% der Bewegungsperiode ruht.

4. Verfahren nach einem der vorangehenden Ansprüche,
bei dem die zyklische Bewegung überwacht wird und bei dem ein Durchgang nur während eines Ruhens der Bewegung gestartet wird oder
bei dem die zyklische Bewegung überwacht wird und bei dem ein Durchgang nur während eines Ruhens der Bewegung gestartet wird und bei dem der Verlauf der zyklischen Bewegung auch prognostiziert wird und bei dem ein Durchgang nur während eines Ruhens der Bewegung gestartet wird, wenn die Prognose erwarten lässt, dass der Durchgang während dieser Zeit zumindest zu 80% abgeschlossen ist.

5. Verfahren nach einem der vorangehenden Ansprüche,
bei dem die Abfolge des Anfahrens der Rasterpunkte von einem der Durchgänge zu einem anderen der Durchgänge verändert wird und/oder
bei dem während der Bestrahlung über eine Mitteilungseinrichtung (120) Informationen betreffend die zyklische Bewegung angegeben werden und/oder
bei dem der Beginn zumindest eines Teils der Durchgänge nach einer Zeitverzögerung zufälliger Dauer erfolgt.

6. Verfahren nach einem der vorangehenden Ansprüche,
bei dem das Zielgebiet (102) ein Zielvolumen (102) ist und die Solldosisverteilung und das Zielraster räumlich ausgestaltet sind oder
bei dem das Zielgebiet (102) ein Zielvolumen (102) ist und die Solldosisverteilung und das Zielraster räumlich ausgestaltet sind und bei dem ein erstes Teilvolumen des Zielvolumens in mehr Durchgängen bestrahlt wird als ein zweites Teilvolumen.

7. Verfahren nach Anspruch 6, bei dem die Anzahl der Durchgänge für zumindest einen Teil der Rasterpunkte unterschiedlich ist.

8. Verfahren nach einem der Ansprüche 6 bis 7, bei dem das räumliche Zielraster schichtweise abgetastet wird und die Anzahl der Durchgänge für zumindest einen Teil der Schichten unterschiedlich ist und/oder
bei dem das räumliche Zielraster schichtweise abgetastet wird und bei dem der Beginn des Bestrahlens für zumindest einen Teil der Schichten in einem der Durchgänge mit einer zufälligen Zeitverzögerung erfolgt und/oder
bei dem das räumliche Zielraster schichtweise abgetastet wird und bei dem der Pfad in einer Schicht von einem der Durchgänge zu einem anderen der Durchgänge verändert wird und/oder
bei dem das räumliche Zielraster schichtweise abgetastet wird und bei dem der Pfad von einer ersten der Schichten zu einer zweiten der Schichten innerhalb desselben Durchgangs geändert wird.

9. Verfahren nach einem der Ansprüche 6 bis 7, bei dem der Strahl einen durch ein Volumen verlaufenden Pfad abfahrt und der Pfad von Durchgang zu Durchgang verändert wird.

10. Verfahren nach einem der vorangehenden Ansprüche,
bei dem durch Modulieren der Intensität des Strahls die Strahlgeschwindigkeit moduliert wird und/oder
bei dem die Mehrfachbestrahlung auf mehrere Sitzungen verteilt und von Sitzung zu Sitzung anders ausgestaltet wird.

11. Bestrahlungsvorrichtung zur Deposition einer Solldosisverteilung in einem zyklisch bewegten Zielgebiet (102) durch Mehrfachbestrahlung mit
einer Strahlerzeugungsvorrichtung (104) und einer Steuerungsvorrichtung (106,108, 110, 112, 114, 116) welche Stahlerzeugungsvorrichtung (104) und Steuerungsvorrichtung (106,108) dazu ausgelegt sind,
mit einem Strahl (105) Rasterpunkte eines Zielrasters in einer Anzahl von zumindest zwei Durchgängen anzufahren, wobei in jedem der Durchgänge Rasterpunkte aufeinanderfolgend angefahren werden,
**dadurch gekennzeichnet, dass**
die Steuerungsvorrichtung dazu ausgelegt ist, den Ablauf der Bestrahlung und die zyklische Bewegung des Zielgebiets (102) zu desynchronisieren mittels Veränderung zumindest einer der folgenden Steuerungsparameter:
- Abtastgeschwindigkeit des Strahls,
- zeitliche Verteilung der Durchgänge, wobei die Durchgänge zufällig verteilt werden oder wobei Pausenzeiten zwischen einzelnen Durchgängen variiert werden,
- zeitliche Verteilung des Bestrahlens von Schichten,
- zeitliche Verteilung des Bestrahlens von bestimmten Volumina innerhalb der einzelnen Durchgänge,
- Anfangsphase der zyklischen Bewegung, wobei über eine Mitteilungseinrichtung einer Person Informationen betreffend die zyklische Bewegung angegeben werden,
- Zyklusdauer der zyklischen Bewegung, wobei die Dauer des Zyklus zufällig geändert wird,
- Pfad des Strahls im Zielgebiet,
und dazu ausgelegt ist, die Bestrahlung des Zielgebiets auf bis zu 30 Durchgänge aufzuteilen,
so dass lokale Fehldosierungen klein gehalten werden.

12. Bestrahlungsvorrichtung nach Anspruch 11,
die ausgebildet ist, zumindest einen Teil der Rasterpunkte in unterschiedlichen Durchgängen gezielt in unterschiedlichen Phasen der zyklischen Bewegung anzufahren und/oder
die ausgebildet ist, die Bewegungsperiode zu bestimmen und zumindest einen Teil der Durchgänge über eine Zeit zu verteilen, die zumindest 50% einer Bewegungsperiode entspricht und/oder
die ausgebildet ist, die Abfolge des Anfahrens der Rasterpunkte von einem der Durchgänge zu einem anderen der Durchgänge zu verändern und/oder
die derart ausbildet ist, dass der Beginn zumindest eines Teils der Durchgänge nach einer Zeitverzögerung zufälliger Dauer erfolgen kann und/oder
die derart ausgebildet ist, dass bei einem Zielvolumen (102) als Zielgebiet die Solldosisverteilung und das Zielraster räumlich ausgestaltet ist.

13. Verfahren zur Bestimmung von Steuerungsparametern für eine Bestrahlungsvorrichtung nach einem der Ansprüche 11 oder 12 zur Durchführung eines Verfahrens zur Deposition einer Solldosisverteilung in einem zyklisch bewegten Zielgebiet (102) durch Mehrfachbestrahlung mit einem Rasterpunkte eines Zielrasters anfahrenden Strahl (105) in einer Anzahl von zumindest zwei Durchgängen, bei dem in jedem der Durchgänge Rasterpunkte aufeinanderfolgend angefahren werden,
**gekennzeichnet durch** die Schritte:
Bestimmen der Vorgehensweise für die Desynchronisierung des Ablaufs der Bestrahlung und der zyklischen Bewegung des Zielgebiets (102), wobei zumindest einer der folgenden Steuerungsparameter:
- Abtastgeschwindigkeit des Strahls,
- zeitliche Verteilung der Durchgänge, wobei die Durchgänge zufällig verteilt werden oder wobei Pausenzeiten zwischen einzelnen Durchgängen variiert werden,
- zeitliche Verteilung des Bestrahlens von Schichten,
- zeitliche Verteilung des Bestrahlens von bestimmten Volumina innerhalb der einzelnen Durchgänge,
- Zyklusdauer der zyklischen Bewegung, wobei die Dauer des Zyklus zufällig geändert wird,
- Pfad des Strahls im Zielgebiet
zur Desynchronisation des Ablaufs der Bestrahlung von der zyklischen Bewegung des Zielgebiets verändert wird, und Aufteilung der Bestrahlung des Zielgebiets auf bis zu 30 Durchgänge,
so dass lokale Fehldosierungen klein gehalten werden.

14. Verwendung einer Bestimmung der Steuerparameter nach Anspruch 13 zur Durchführung eines Verfahrens nach einem der Ansprüche 1 bis 10.

## Claims

1. A method for depositing an intended dose distribution in a cyclically moved target region (102), not located in a human or animal, by multiple irradiations with a beam (105) that approaches grid points of a target grid in a number of at least two passes, wherein grid points are approached sequentially in each of the passes; **characterized by** the steps of:
de-synchronizing the irradiation process and the cyclical movement of the target region (102) by altering at least one of the following control parameters:
- scanning velocity of the beam;
- time distribution of the passes, wherein the passes are randomized or pause durations between individual passes are varied;
- time distribution of the irradiation of layers;
- time distribution of the irradiation of particular volumes during the individual passes;
- cycle duration of the cyclical movement, wherein the duration of the cycle is altered randomly;
- path of the beam in the target region; and
partitioning the irradiation of the target region into up to 30 passes so that localized incorrect dosages are kept small.

2. The method according to claim 1,
wherein in different passes at least part of the grid points is selectively approached in different phases of the cyclical movement; or
wherein at least part of the grid points is selectively approached in different phases of the cyclical movement and wherein the cyclical movement is monitored.

3. The method according to any one of the preceding claims,
wherein the period of movement is determined and at least part of the passes is distributed over a time corresponding to at least 50 % of a period of movement; or
wherein the period of movement is determined and at least part of the passes is distributed over a time corresponding to at least 50 % of a period of movement, and wherein the period of movement is determined prior to the irradiation; or
wherein the period of movement is determined and at least part of the passes is distributed over a time corresponding to at least 50 % of a period of movement, and wherein the period of movement is determined multiple times during the irradiation by monitoring the movement; or
wherein the period of movement is determined and at least part of the passes is distributed over a time corresponding to at least 50 % of a period of movement, and wherein the period of movement is determined prior to the irradiation, and wherein the period of movement is determined multiple times during the irradiation by monitoring the movement; and/or
wherein the movement is monitored and the irradiation is stopped when the movement is at rest for more than 50 % of the period of movement.

4. The method according to any one of the preceding claims,
wherein the cyclical movement is monitored and wherein a pass is started only while the movement is at rest; or
wherein the cyclical movement is monitored and wherein a pass is started only while the movement is at rest, and wherein the process of the cyclical movement is furthermore predicted, and wherein a pass is started only while the movement is at rest and if the prediction suggests that the pass is at least 80 % completed during this time.

5. The method according to any one of the preceding claims,
wherein the sequence of approaching the grid points is altered from one of the passes to another one of the passes; and/or
wherein information relating to the cyclical movement is indicated during the irradiation via a notification means (120); and/or
wherein at least part of the passes is started after a time delay of random duration.

6. The method according to any one of the preceding claims,
wherein the target region (102) is a target volume (102) and the target dose distribution and the target grid are configured in three dimensions; or
wherein the target region (102) is a target volume (102) and the target dose distribution and the target grid are configured in three dimensions, and wherein a first partial volume of the target volume is irradiated in more passes than a second partial volume.

7. The method according to claim 6, wherein the number of passes is different for at least part of the grid points.

8. The method according to any one of claims 6 to 7,
wherein the three-dimensional target grid is scanned layer by layer, and wherein the number of passes is different for at least part of the layers; and/or
wherein the three-dimensional target grid is scanned layer by layer, and wherein in one of the passes the irradiation is started with a random time delay for at least part of the layers; and/or
wherein the three-dimensional target grid is scanned layer by layer, and wherein the path within a layer is altered from one of the passes to another one of the passes; and/or
wherein the three-dimensional target grid is scanned layer by layer, and wherein during the same pass the path is altered from a first one of the layers to a second one of the layers.

9. The method according to any one of claims 6 to 7, wherein the beam is moved through a volume along a path and the path is altered from one pass to another pass.

10. The method according to any one of the preceding claims,
wherein the velocity of the beam is modulated by modulating the intensity of the beam; and/or
wherein the multiple irradiations are distributed to a plurality of sessions and configured differently from session to session.

11. An irradiation device for depositing an intended dose distribution in a cyclically moved target region (102) by multiple irradiations using a beam generating device (104) and a control device (106, 108, 110, 112, 114, 116), said beam generating device (104) and control device (106,108) being adapted to
approach, with a beam (105), grid points of a target grid in a number of at least two passes, the grid points being approached sequentially in each of the passes; **characterized in that**
the control device is adapted to de-synchronize the irradiation process and the cyclical movement of the target region (102) by altering at least one of the following control parameters:
- scanning velocity of the beam;
- time distribution of the passes, wherein the passes are randomized or pause durations between individual passes are varied;
- time distribution of the irradiation of layers;
- time distribution of the irradiation of particular volumes during the individual passes;
- starting phase of the cyclical movement, wherein information relating to the cyclical movement is notified to a person via a notification means;
- cycle duration of the cyclical movement, wherein the duration of the cycle is altered randomly;
- path of the beam in the target region;
and is adapted to partition the irradiation of the target region into up to 30 passes, so that localized incorrect dosages are kept small.

12. The irradiation device according to claim 11,
adapted to selectively approach, in different passes, at least part of the grid points in different phases of the cyclical movement; and/or
adapted to determine the period of movement and to distribute at least part of the passes over a time corresponding to at least 50 % of a period of movement; and/or
adapted to alter the sequence of approaching the grid points from one of the passes to another one of the passes; and/or
adapted to allow at least part of the passes to start after a time delay of random duration; and/or
adapted so that in a target region in the form of a target volume (102) the target dose distribution and the target grid are configured in three dimensions.

13. A method for determining control parameters for an irradiation device according to claim 11 or 12 for carrying out a method for depositing an intended dose distribution in a cyclically moved target region (102) by multiple irradiations with a beam (105) that approaches grid points of a target grid in a number of at least two passes, wherein grid points are approached sequentially in each of the passes;
**characterized by** the steps of:
determining the procedure for de-synchronizing the irradiation process and the cyclical movement of the target region (102), by altering at least one of the following control parameters:
- scanning velocity of the beam;
- time distribution of the passes, wherein the passes are randomized or pause durations between individual passes are varied;
- time distribution of the irradiation of layers;
- time distribution of the irradiation of particular volumes during the individual passes;
- cycle duration of the cyclical movement, wherein the duration of the cycle is altered randomly;
- path of the beam in the target region;
for de-synchronizing the irradiation process and the cyclical movement of the target region; and
partitioning the irradiation of the target region into up to 30 passes,
so that localized incorrect dosages are kept small.

14. Use of a determination of the control parameters according to claim 13 for carrying out a method according to any one of claims 1 to 10.

## Revendications

1. Procédé de dépôt d'une distribution de doses de consigne dans une zone cible (102) présentant des mouvements cycliques, qui n'est pas située dans un être humain ou un animal, par irradiations multiples avec un faisceau (105) dirigé sur des points de trame d'une trame cible, au cours d'au moins deux passages, procédé lors duquel des points de trame sont visés successivement au cours de chacun des passages, **caractérisé en ce qu'**il comporte les étapes suivantes :
désynchronisation du déroulement de l'irradiation et du mouvement cyclique de la zone cible (102), par modification d'au moins un des paramètres de commande suivants :
- vitesse de balayage du faisceau,
- répartition des passages dans le temps, sachant que les passages sont répartis de façon aléatoire ou que l'on fait varier les temps de pause entre les différents passages,
- répartition dans le temps de l'irradiation de couches,
- répartition dans le temps de l'irradiation de volumes définis, au cours des différents passages,
- durée de cycle du mouvement cyclique, avec modification aléatoire de la durée du cycle,
- trajectoire du faisceau dans la zone cible, et
répartition de l'irradiation de la zone cible sur jusqu'à 30 passages, de façon à maintenir les erreurs de dosage locales à un niveau faible.

2. Procédé selon la revendication 1,
selon lequel, au cours de différents passages, au moins une partie des points de trame est visée de manière ciblée dans des phases différentes du mouvement cyclique, ou
selon lequel, au cours de différents passages, au moins une partie des points de trame est visée de manière ciblée dans des phases différentes du mouvement cyclique et lors duquel le mouvement cyclique est surveillé.

3. Procédé selon l'une des revendications précédentes,
selon lequel on détermine la période de mouvement et on répartit au moins une partie des passages sur une durée qui correspond à au moins 50 % d'une période de mouvement, ou
selon lequel on détermine la période de mouvement et on répartit au moins une partie des passages sur une durée qui correspond à au moins 50 % d'une période de mouvement, et selon lequel la période de mouvement est déterminée avant l'irradiation, ou
selon lequel on détermine la période de mouvement et on répartit au moins une partie des passages sur une durée qui correspond à au moins 50 % d'une période de mouvement, et selon lequel la période de mouvement est déterminée plusieurs fois pendant l'irradiation, en surveillant le mouvement, ou
selon lequel on détermine la période de mouvement et on répartit au moins une partie des passages sur une durée qui correspond à au moins 50 % d'une période de mouvement, et selon lequel la période de mouvement est déterminée avant l'irradiation, et selon lequel la période de mouvement est déterminée plusieurs fois pendant l'irradiation, en surveillant le mouvement, et/ou
selon lequel on surveille le mouvement et on interrompt l'irradiation lorsque le mouvement s'arrête pendant plus de 50 % de la période de mouvement.

4. Procédé selon l'une des revendications précédentes,
selon lequel on surveille le mouvement cyclique et selon lequel on démarre un passage uniquement pendant un arrêt du mouvement, ou
selon lequel on surveille le mouvement cyclique et selon lequel on démarre un passage uniquement pendant un arrêt du mouvement, et selon lequel le déroulement du mouvement cyclique est également pronostiqué, et selon lequel on démarre un passage uniquement pendant un arrêt du mouvement, si d'après le pronostic on peut s'attendre à ce que le passage soit achevé à au moins 80 % pendant ce temps.

5. Procédé selon l'une des revendications précédentes,
selon lequel l'ordre de sélection des points de trame est modifié de l'un des passages à un autre des passages, et/ou
selon lequel, au cours de l'irradiation, un dispositif de notification (120) fournit des informations relatives au mouvement cyclique, et/ou
selon lequel le début d'au moins une partie des passages a lieu après une temporisation de durée aléatoire.

6. Procédé selon l'une des revendications précédentes,
selon lequel la zone cible (102) est un volume cible (102) et la distribution de doses de consigne et la trame cible sont réalisées de façon tridimensionnelle, ou
selon lequel la zone cible (102) est un volume cible (102) et la distribution de doses de consigne et la trame cible sont réalisées de façon tridimensionnelle, et selon lequel un premier volume partiel du volume cible est irradié pendant un nombre de passages qui est plus élevé que celui pour un deuxième volume partiel.

7. Procédé selon la revendication 6, selon lequel le nombre de passages est différent au moins pour une partie des points de trame.

8. Procédé selon l'une des revendications 6 à 7, selon lequel la trame cible tridimensionnelle est balayée par couches et le nombre de passages est différent pour au moins une partie des couches, et/ou
selon lequel la trame cible tridimensionnelle est balayée par couches, et selon lequel le début de l'irradiation s'effectue pour un des passages avec une temporisation aléatoire, au moins pour une partie des couches, et/ou
selon lequel la trame cible tridimensionnelle est balayée par couches, et selon lequel on modifie la trajectoire dans une couche, d'un passage à un autre passage, et/ou
selon lequel la trame cible tridimensionnelle est balayée par couches, et selon lequel on modifie la trajectoire d'une première des couches vers une deuxième des couches, au cours du même passage.

9. Procédé selon l'une des revendications 6 à 7, selon lequel le faisceau suit une trajectoire passant par un volume, et la trajectoire est modifiée d'un passage à l'autre.

10. Procédé selon l'une des revendications précédentes,
selon lequel la vitesse du faisceau est modulée par modulation de l'intensité du faisceau, et/ou
selon lequel les irradiations multiples sont réparties sur plusieurs séances et sont réalisées de manière différente d'une séance à l'autre.

11. Dispositif d'irradiation destiné au dépôt d'une distribution de doses de consigne dans une zone cible (102) présentant des mouvements cycliques, par irradiations multiples avec un dispositif de production de faisceaux (104) et un dispositif de commande (106, 108, 110. 112, 114, 116), lesquels dispositif de production de faisceaux (104) et dispositif de commande (106, 108) sont conçus pour
diriger un faisceau (105) sur des points de trame d'une trame cible, au cours d'au moins deux passages, sachant que lors de chacun des passages, des points de trame sont visés successivement,
**caractérisé en ce que**
le dispositif de commande est conçu pour désynchroniser le déroulement de l'irradiation et le mouvement cyclique de la zone cible (102), par modification d'au moins un des paramètres de commande suivants :
- vitesse de balayage du faisceau,
- répartition des passages dans le temps, sachant que les passages sont répartis de façon aléatoire ou que l'on fait varier les temps de pause entre les différents passages,
- répartition dans le temps de l'irradiation de couches,
- répartition dans le temps de l'irradiation de volumes définis, au cours des différents passages,
- phase initiale du mouvement cyclique, lors de laquelle un dispositif de notification fournit à une personne des informations relatives au mouvement cyclique,
- durée de cycle du mouvement cyclique, avec modification aléatoire de la durée du cycle,
- trajectoire du faisceau dans la zone cible, et
et est conçu pour répartir l'irradiation de la zone cible sur jusqu'à 30 passages, de façon à maintenir les erreurs de dosage locales à un niveau faible.

12. Dispositif d'irradiation selon la revendication 11,
qui est conçu pour viser de manière ciblée au moins une partie des points de trame lors de passages différents, au cours de phases différentes du mouvement cyclique, et/ou
qui est conçu pour déterminer la période de mouvement et répartir au moins une partie des passages sur une durée qui correspond à au moins 50 % d'une période de mouvement, et/ou
qui est conçu pour modifier l'ordre de sélection des points de trame, d'un passage à un autre des passages, et/ou
qui est conçu de manière telle que le début d'au moins une partie des passages puisse avoir lieu après une temporisation de durée aléatoire, et/ou
qui est conçu de manière telle que pour un volume cible (102) en tant que zone cible, la distribution de doses de consigne et la trame cible soient réalisées de façon tridimensionnelle.

13. Procédé de détermination de paramètres de commande pour un dispositif d'irradiation selon l'une des revendications 11 ou 12, en vue de la mise en œuvre d'un procédé de dépôt d'une distribution de doses de consigne dans une zone cible (102) présentant des mouvements cycliques, par irradiations multiples avec un faisceau (105) visant des points de trame d'une trame cible, au cours d'au moins deux passages, procédé lors duquel des points de trame sont sélectionnés successivement au cours de chacun des passages,
**caractérisé en ce qu'**il comporte les étapes suivantes :
détermination du mode opératoire pour la désynchronisation du déroulement de l'irradiation et du mouvement cyclique du volume cible (102), où l'on modifie au moins un des paramètres de commande suivants :
- vitesse de balayage du faisceau,
- répartition des passages dans le temps, sachant que les passages sont répartis de façon aléatoire ou que l'on fait varier les temps de pause entre les différents passages,
- répartition dans le temps de l'irradiation de couches,
- répartition dans le temps de l'irradiation de volumes définis, au cours des différents passages,
- durée de cycle du mouvement cyclique, avec modification aléatoire de la durée du cycle,
- trajectoire du faisceau dans la zone cible,
en vue de la désynchronisation du déroulement de l'irradiation par rapport au mouvement cyclique de la zone cible, et en vue de la répartition de l'irradiation de la zone cible sur jusqu'à 30 passages, de façon à maintenir les erreurs de dosage locales à un niveau faible.

14. Utilisation d'une détermination des paramètres de commande selon la revendication 13, à des fins de mise en œuvre d'un procédé selon l'une des revendications 1 à 10.
